# FASCICULE DE BREVET EUROPEEN

(11) **EP 2 662 072 B1**
(45) Date de publication et mention de la délivrance du brevet: **03.01.2018**
(21) Numéro de dépôt: 13305270.4
(22) Date de dépôt: 12.03.2013
(51) Int. Cl.: A61K 8/97, A61Q 19/08, A61K 31/702, A61K 8/64, A61K 31/733, A61K 36/28

(54) **Principe actif obtenu à partir de cichorium intybus pour une action sur la fonction barrière de la peau similaire à celle de la vitamine D**
Aus Cichorium intybus erhaltener Wirkstoff, der eine Vitamin-D-ähnliche Wirkung auf die Barrierefunktion der Haut ausüben soll
Active ingredient obtained from Cichorium intybus for action on the skin barrier function similar to that of vitamin D

(30) Priorité: 12.03.2012 FR 1252183
(43) Date de publication de la demande: 13.11.2013
(73) Titulaire: Societe Industrielle Limousine d'Application Biologique, 19130 Objat (FR)
(72) Inventeur: Paufique, Jean, 19130 Objat (FR)
(74) Mandataire: Aquinov

(56) Documents cités:
- EP-A1- 0 326 491
- WO-A2-2005/053710
- JP-A- 63 309 147
- Jurlique: "Night Cream - Jurlique Herbal Recovery", Mintel, 1 février 2011 (2011-02-01), XP055049025, Extrait de l'Internet: URL:http://www.gnpd.com/sinatra/gnpd/searc h_results&search_id=lsNmzYB2P9/&p_page_num ber=2&item_id=1494381 [extrait le 2013-01-09]

## Description

La présente invention se rapporte à un principe actif particulier issu de racine de *Cichorium intybus* et son utilisation sur la peau dans une composition à application topique, pour une action similaire à celle de la vitamine D.

L'invention concerne également les compositions cosmétiques incluant de telles molécules, et un procédé de traitement cosmétique destiné à préserver ou accélérer la récupération de la fonction barrière de la peau.

La vitamine D est bien connue pour son rôle primordial dans le contrôle de l'homéostasie phosphocalcique et dans la minéralisation osseuse. La synthèse de vitamine D est initiée au niveau de la peau sous l'action des UVB et sa forme biologiquement active, le calcitriol est ensuite produite dans le rein pour exercer ses fonctions.

Néanmoins, depuis peu, on sait qu'il existe également un système cutané autonome pour la production de calcitriol au niveau de la peau, et la découverte de ce système a permis de révéler l'implication de la vitamine D dans d'autres fonctions cellulaires cutanées : contrôle de la croissance et différenciation cellulaire, rôle cytoprotecteur et immunomodulateur.

La production locale de calcitriol au niveau de la peau résulte de plusieurs étapes successives initiées au niveau de l'épiderme qui contient son précurseur, la provitamine D. Sous l'action des UVB, la provitamine D est convertie en pré-vitamine D3, elle-même transformée en vitamine D3 qui est ensuite activée au niveau des kératinocytes en calcitriol. La vitamine D ainsi activée est capable d'exercer des effets autocrines et paracrines par l'intermédiaire d'un récepteur, le récepteur à la vitamine D (VDR). Par cette voie, la vitamine D régule de nombreux processus impliqués dans l'homéostasie cutanée : la formation et le maintien de la barrière épidermique, la croissance capillaire, le système immunitaire inné ainsi que le vieillissement.

La synthèse de vitamine D est sous l'influence de nombreux facteurs : internes comme l'âge ou le phototype, externes comme la saison, la pollution de l'air ou l'intensité des radiations UV, ou encore comportementaux comme le port de vêtements, l'utilisation d'écrans solaires, etc. De plus, on sait que l'insuffisance en vitamine D est très fréquente et en constante augmentation, en particulier chez les personnes âgées et les femmes qui sont des populations plus à risque.

L'objectif de la présente invention est de proposer un principe actif qui favorise la capacité de la peau, en particulier des peaux âgées, à stimuler les voies de signalisation régulée par le VDR en agissant comme la vitamine D.

A cet effet, l'invention vise l'utilisation de molécules particulières obtenues à partir de racine de *Cichorium intybus.*

La chicorée amère ou chicorée sauvage ou *Cichorium intybus* est une plante herbacée de la famille des Astéracées. Fréquente sur toutes les parcelles, elle est facilement identifiée par ses capitules terminaux et axillaires de fleurs bleues toutes ligulées. Elle est originaire d'Europe, des régions tempérées d'Asie et d'Afrique du Nord, et naturalisée en Amérique du Nord.

La chicorée est largement cultivée pour son usage fourrager et alimentaire. Ses parties aériennes sont consommées en salade. Depuis le milieu du dix-huitième siècle, les racines récoltées à l'automne sont torréfiées pour être utilisés comme succédané du café.

La chicorée est également utilisée traditionnellement pour ses vertus médicinales cholérétique, cholagogue, diurétique, dépurative et digestive.

Des extraits de *Cichorium intybus* ont par ailleurs été utilisés dans des produits cosmétiques, en particulier pour un effet pigmentant dans la demande EP-1.707.191 ou EP-2.277.502, anti-inflammatoire dans la demande EP-1.962.875, anti-radicalaire pour prévenir le vieillissement cutané dans la demande FR-2.626.469, ou encore en mélange avec d'autres plantes dans des produits cosmétiques amincissant ou adoucissant.

La présente invention vise spécifiquement l'utilisation cosmétique d'un hydrolysat de racine de *Cichorium intybus* obtenu par un procédé comprenant une étape d'hydrolyse enzymatique comprenant des oligofructosanes de degré de polymérisation compris entre 3 et 20 comme principe actif dans une composition à application cutanée, pour agir de façon similaire à la vitamine D sur les cellules de la peau, en particulier pour stimuler les voies de signalisation régulées par le récepteur à la vitamine D dans les cellules cutanées.

En effet, de façon surprenante l'utilisation sur la peau d'oligofructosanes obtenus par hydrolyse de racine de *Cichorium intybus* par un procédé comprenant une étape d'hydrolyse enzymatique permet de restaurer la fonctionnalité du VDR dans les cellules de la peau de personnes susceptibles de présenter une déficience en vitamine D, et par ce mécanisme de stimuler le réseau moléculaire impliqué dans la différenciation terminale des kératinocytes et favoriser la formation de la barrière cutanée.

De façon spécifique, l'invention vise également un principe actif cosmétique particulier à savoir un hydrolysat de racine de *Cichorium intybus* comprenant des oligofructosanes de degré de polymérisation compris entre 3 et 20 obtenu par un procédé comprenant une étape d'hydrolyse enzymatique. Par « hydrolysat » on entend tout extrait obtenu à partir de racine de *Cichorium intybus,* comprenant au moins une étape d'hydrolyse enzymatique.

Enfin, l'invention a également pour objet une composition cosmétique contenant ce principe actif, ainsi qu'un procédé de traitement cosmétique destiné à préserver l'intégrité ou favoriser une récupération de la fonction barrière de la peau comprenant l'application topique d'une telle composition.

La présente invention est maintenant décrite en détail, en regard des figures annexées sur lesquelles :
- les figures la à 1d représentent les images obtenues pour les résultats de l'étude A.III sur l'épaisseur de l'épiderme à J19 :
   ∘ la témoin épidermes reconstruits jeunes,
   ∘ 1b témoin épidermes reconstruits vieillis,
   ∘ 1c épidermes reconstruits vieillis + calcitriol 10⁻⁸M,
   ∘ 1d épidermes reconstruits vieillis + principe actif exemple 1 à 0,5%,

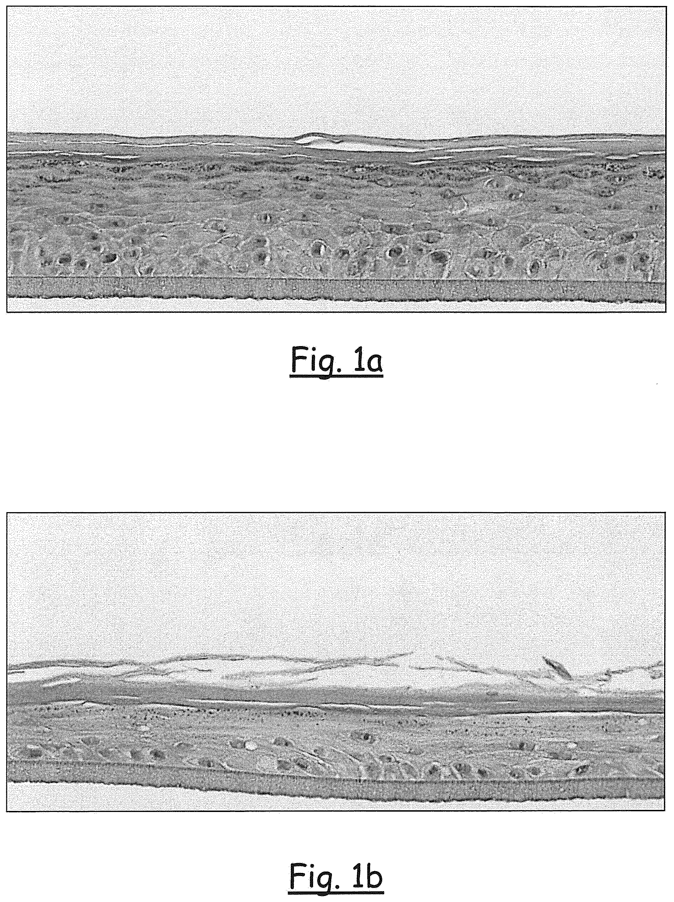

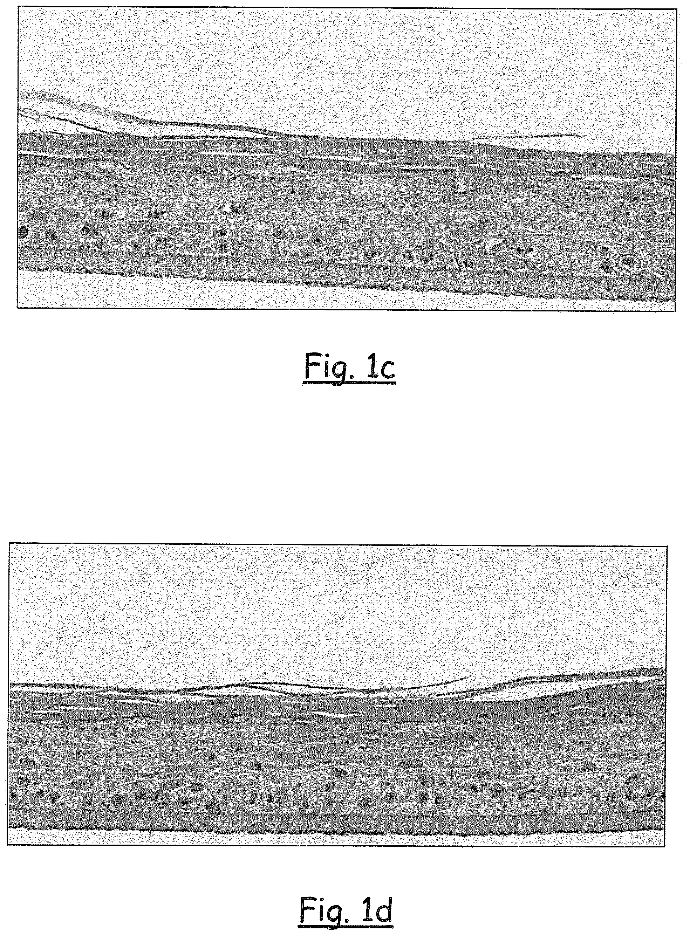

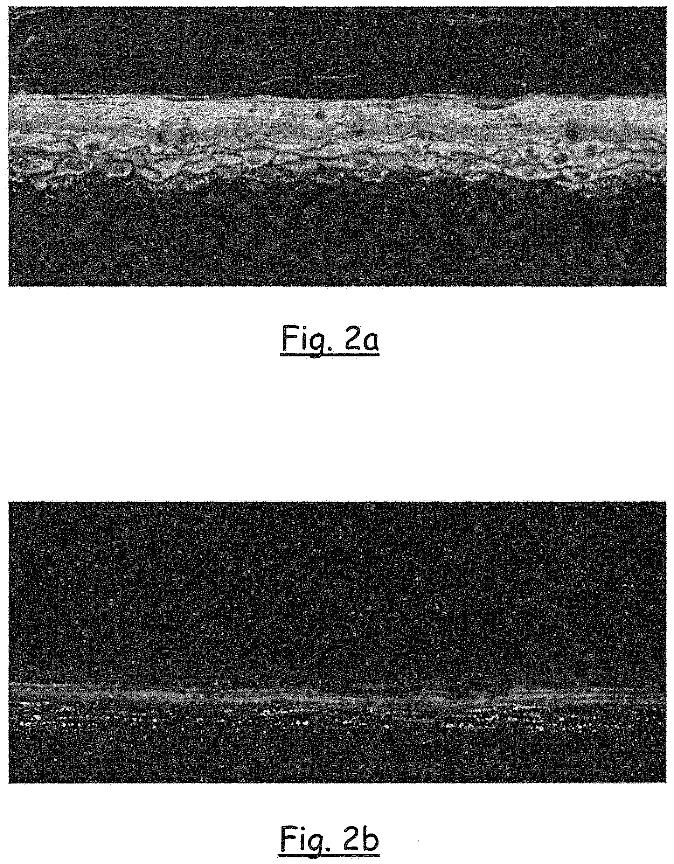

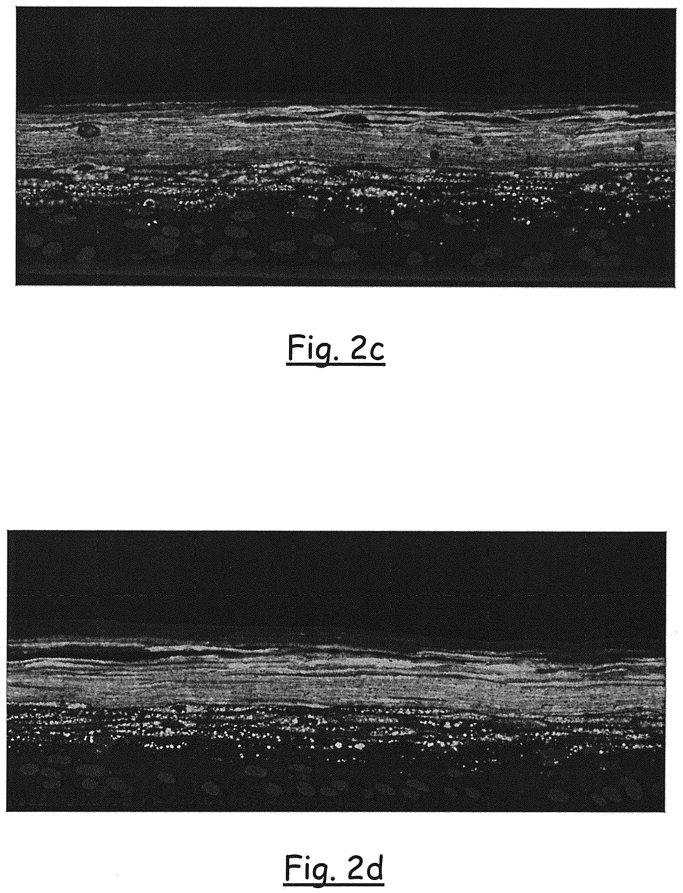

- les figures 2a à 2d représentent les images obtenues pour les résultats de l'étude A.III sur la synthèse de filaggrine à J19 :
   ∘ 2α témoin épidermes reconstruits jeunes,
   ∘ 2b témoin épidermes reconstruits vieillis,
   ∘ 2c épidermes reconstruits vieillis + calcitriol 10⁻⁸M,
   ∘ 2d épidermes reconstruits vieillis + principe actif exemple 1 à 0,5%.

### UTILISATION

Selon un premier aspect, l'invention vise un hydrolysat de racine de *Cichorium intybus* comprenant des oligofructosanes de degré de polymérisation compris entre 3 et 20 obtenu par un procédé comprenant une étape d'hydrolyse enzymatique pour son application comme principe actif dans une composition à application cutanée, ledit principe actif et/ou ladite composition étant destinés à agir de façon similaire à la vitamine D dans les cellules de la peau, en particulier pour un effet restructurant cutané. Appliqués sur la peau, le principe actif et/ou la composition permettent notamment de stimuler la synthèse du récepteur à la vitamine D (VDR) et/ou d'augmenter la fonctionnalité du VDR dans les cellules cutanées et de stimuler le réseau de gènes engagés dans la différenciation terminale des kératinocytes, régulée par le VDR.

Le mode d'action de la vitamine D le mieux connu implique l'activation de son récepteur nucléaire spécifique, le VDR, qui régule l'expression de gènes cibles.

Au niveau cutané, le VDR est exprimé par les kératinocytes, les fibroblastes, les mélanocytes, les cellules de langerhans, les macrophages ainsi que les cellules endothéliales, ce qui contribue aux effets cellulaires variés de la vitamine D.

Le taux de VDR est un paramètre important dans la réponse biologique de la vitamine b. Plus il est élevé, plus l'activité transcriptionnelle des gènes cibles activés est haute.

Selon l'invention, l'utilisation sur la peau d'un hydrolysat de racine de *Cichorium intybus* comprenant des oligofructosanes de degré de polymérisation compris entre 3 et 20 obtenu par un procédé comprenant une étape d'hydrolyse enzymatique, permet de stimuler la synthèse du récepteur à la vitamine D (VDR) et/ou augmenter la fonctionnalité du VDR dans les cellules cutanées, agissant ainsi de façon similaire à la vitamine D.

On sait en outre que la vitamine D et son récepteur régulent de nombreux processus centraux impliqués dans l'homéostasie de l'épiderme, en particulier les étapes clés qui aboutissent à la formation d'une fonction barrière optimale et fonctionnelle.

L'épiderme est un épithélium pluristratifié composé de cellules ayant une morphologie spécifique à chaque couche. L'épiderme se renouvelle par divisions cellulaires à partir des cellules basales puis par différenciation des kératinocytes en cornéocytes. Au cours de leur migration vers la surface de la peau, les kératinocytes subissent des modifications biochimiques et structurales, pour se transformer progressivement en cornéocytes. La différenciation terminale du cornéocyte implique la formation d'une matrice fibreuse intracornéocytaire, la production de lipides intercellulaires et l'apparition d'une enveloppe cornée conduisant à la formation du *stratum corneum.* Les cornéocytes sont finalement éliminés grâce au processus de desquamation qui permet le renouvellement de l'épiderme et le maintien de son épaisseur.

Les trois étapes de différenciation, de cornification et de desquamation sont essentielles à l'intégrité de l'épiderme et à la formation de sa barrière fonctionnelle. Le VDR est au centre du réseau de gènes qui régulent ces étapes :
- il stimule le Kruppel-Like Factor 4 (KLF4 facteur de type Kruppel 4), facteur de transcription qui orchestre le développement de la barrière épidermique en contrôlant l'expression des marqueurs de différenciation terminale,
- il augmente l'expression des cytokératines 1 et 10 ainsi que celle de l'involucrine et de la transglutaminase et accélère ainsi le processus de différenciation et de maturation de l'enveloppe cornée,
- il contrôle l'expression des inhibiteurs de protéases comme la cystatine E/M. La cystatine E/M, inhibiteur des cystéines protéases lysosomales, intervient d'une part dans la différenciation terminale des kératinocytes en inhibant indirectement l'activité des transglutaminases qui permettent la liaison des protéines structurales de l'enveloppe cornée. Elle contrôle d'autre part le processus de desquamation en limitant l'activité de la cathepsine V.
- il stimule l'expression des kallikréines (KLK) qui sont des sérine protéases impliquées dans le processus de desquamation, en particulier les KLK5 ou KLK7 qui sont responsables de la dégradation des liens intercornéocytaires composés de la desmogléine-1, la desmocolline-1 ainsi que de la cornéodesmosine.

Selon l'invention, l'utilisation sur la peau d'un hydrolysat de racine de *Cichorium intybus* comprenant des oligofructosanes de degré de polymérisation compris entre 3 et 20 obtenu par un procédé comprenant une étape d'hydrolyse enzymatique, permet de stimuler les voies de signalisation régulées par le récepteur à la vitamine D dans les cellules cutanées, en particulier d'augmenter l'expression de KLF4, de la cytokératine 1, de l'involucrine, de la cystatine E/M et/ou de la KLK5. Il stimule ainsi le réseau de gènes engagés dans la différenciation terminale des kératinocytes et favorise la formation de la barrière cutanée.

L'invention est particulièrement adaptée aux peaux susceptibles de présenter une déficience en vitamine D, en particulier les peaux âgées. On sait en effet que le taux de vitamine D dans la peau varie et dépend de nombreux facteurs. En particulier, plusieurs études ont montré un déclin relatif à l'âge de la synthèse cutanée de vitamine D et des réponses tissulaires induites.

De plus, la demanderesse a démontré que les kératinocytes humains âgés présentent une capacité réduite à synthétiser le récepteur à la vitamine D (VDR) en comparaison à des kératinocytes humains jeunes.

Aussi, en agissant de façon similaire à la vitamine D, un hydrolysat enzymatique de racine de *Cichorium intybus* comprenant des oligofructosanes de degré de polymérisation compris entre 3 et 20 appliqué sur la peau, permet de restaurer la capacité endogène des cellules âgées à stimuler les voies de signalisation régulée par le VDR. Il renforce ainsi la barrière cutanée et améliore la capacité de récupération de la peau de personnes matures, susceptibles de présenter une déficience en vitamine D.

Selon un mode de réalisation particulièrement adapté, l'invention vise l'utilisation d'un hydrolysat de racine de *Cichorium intybus* comprenant des oligofructosanes de degré de polymérisation compris entre 3 et 20 obtenu par un procédé comprenant une étape d'hydrolyse enzymatique tel que décrit en suivant.

### PRINCIPE ACTIF

L'invention concerne également un principe actif cosmétique particulier, à savoir un hydrolysat de racine de *Cichorium intybus* comprenant des o ligofructosanes de degré de polymérisation compris entre 3 et 20 obtenu par un procédé comprenant une étape d'hydrolyse enzymatique. Selon un mode de réalisation particulièrement adapté :
- les sucres totaux représentent au moins 46% en poids de la matière sèche du principe actif, et/ou
- les oligofructosanes de degré de polymérisation compris entre 3 et 20 représentent au moins 69% en poids des sucres totaux du principe actif.

De façon préférée les oligofructosanes de degré de polymérisation compris entre 3 et 20 représentent au moins 31% en poids de matière sèche du principe actif.

Le principe actif selon l'invention est un hydrolysat enzymatique.

Le principe actif présente préférentiellement une couleur jaune clair.

Il peut se présenter sous forme liquide et être défini par au moins une des caractéristiques exposées ci-après, préférentiellement toutes.

### Matières sèches :

Le taux de matières sèches d'un principe actif selon l'invention (mesuré par passage à l'étuve à 105°C en présence de sable d'un échantillon de poids initial donné jusqu'à obtention d'un poids constant) peut être compris entre 30 et 90 g/l, préférentiellement entre 45 et 65 g/l.

### Mesure du pH :

Le pH (mesuré par la méthode potentiométrique à température ambiante) peut être compris entre 3,5 et 6,5, préférentiellement entre 4,5 et 5,5.

### Carbohydrates :

### Détermination de la teneur en sucres totaux

Le dosage de la teneur en sucres totaux peut être réalisé par la méthode de DUBOIS (DUBOIS M. et al., (1956), Analytical chemistry, 28, n°3 p. 350-356). En présence d'acide sulfurique concentré et de phénol, les sucres réducteurs donnent un composé jaune orangé. A partir d'une gamme étalon, on peut déterminer le taux de sucres totaux d'un échantillon. La teneur en sucres totaux peut être comprise entre 20 et 62 g/l, préférentiellement entre 30 et 45g/l.

La teneur en sucres totaux est préférentiellement supérieure ou égale à 46% en poids de matière sèche.

### Caractérisation de la fraction carbohydrate :

La détermination de la taille des carbohydrates d'un principe actif selon l'invention est réalisée par chromatographie liquide haute performance.

Le chromatogramme obtenu montre la présence entre 10 et 31% de monosaccharides de masse moléculaire inférieure à 180Da et entre 69 et 90% d'oligosaccharides de masse molaire comprise entre 180 et 5900Da (degré de polymérisation maximum de 33).

La caractérisation des carbohydrates du principe actif par chromatographie CLHP, montre que les sucres du principe actif selon l'invention sont pour au moins 69% des oligosaccharides de degré de polymérisation compris entre 3 et 20.

Par ailleurs, l'analyse de la composition en sucres simples du principe actif selon l'invention, montre qu'il est composé essentiellement de fructose sous forme liée.

La fraction glucidique du principe actif selon l'invention est donc composée essentiellement d'oligofructosanes de degré de polymérisation compris entre 3 et 20.

### Teneur en cendres brutes :

La teneur en cendres brutes est déterminée par la pesée des résidus issus de l'incinération à 550°C dans un four à moufle électrique (VULCAN™).

Le poids du résidu est calculé en déduisant la tare.

La teneur en minéraux est exprimée en pourcentage par rapport au poids total de la matière sèche du principe actif.

La teneur en cendres brutes d'un principe actif selon l'invention est préférentiellement comprise entre 7,9 et 9,7%.

### Teneur en acides uroniques :

Le produit de l'acide galacturonique avec le tétraborate de sodium, donne en présence du méta-hydroxydiphényl, une coloration rose permettant le dosage au spectrophotomètre à 520 nm.

L'intensité de coloration est proportionnelle à la quantité d'acides uroniques.

Les lectures sont réalisées à partir d'une gamme étalon d'acide galacturonique allant de 10 à 100 mg/l.

Les résultats obtenus pour les étalons permettent de tracer une droite densité optique en fonction de la concentration et le taux d'acides uroniques des échantillons est lu directement sur cette droite.

La teneur en acides uroniques d'un principe actif selon l'invention peut être comprise entre 9,3 et 11,5 % en poids par rapport à la matière sèche.

### Protéines :

La teneur en azote total est déterminée par la méthode de KJELDHAL, elle représente la teneur en protéines.

La teneur en protéines d'un principe actif selon l'invention est préférentiellement comprise entre 4,2 et 5,2% en poids par rapport à la matière sèche.

Par ailleurs, la caractérisation de la fraction protéique du principe actif selon l'invention réalisée par FPLC montre qu'elle est majoritairement constituée de peptides ayant une masse moléculaire inférieure à 2000Da.

### Composés phénoliques :

Le dosage des composés phénoliques est réalisé par lecture de l'intensité de coloration en présence de ferricyanure de potassium, intensité détectable à 715 nm et comparée à une gamme étalon d'acide gallique.

Le taux de polyphénols est inférieur à 0,05% de la matière sèche.

Le principe actif selon l'invention ne contient pas de composés polyphénoliques.

### Identification de la fraction active

Afin de démontrer que la fraction majoritairement active du principe actif selon l'invention est bien la fraction constituée par les oligofructosanes de degré de polymérisation compris entre 3 et 20, une étude a été réalisée. Cette étude consiste à fractionner les principales espèces moléculaires du principe actif selon l'invention :
- une fraction A constituée de cendres, obtenue par ressolubilisation des résidus issus de l'incinération à 550°C dans un four à moufle électronique, reprise dans de l'eau distillée et filtrée,
- une fraction B contenant 98% des carbohydrates du principe actif, et une très faible quantité de protéines.

L'étude consiste à comparer l'effet de ces différentes fractions sur l'expression de l'involucrine et de la KLK5 par PCR quantitative sur kératinocytes humains normaux, au résultat obtenu pour le principe actif selon l'invention à 1%. Le protocole de l'essai est celui décrit au point II.

Les résultats obtenus sont présentés dans le tableau ci-après :

| | **Expression d'ARNm (%)** | |
|---|---|---|
| | **Involucrine** | **KLK5** |
| Témoin | 100 | 100 |
| Principe actif exemple 1 1% | 151 | 122 |
| Fraction A 1% | 68 | 69 |
| Fraction B 1% | 138 | 126 |

La fraction A n'est pas efficace. C'est la fraction B qui confère au principe actif son activité.

L'analyse de la fraction B par HPLC (Chromatographie Liquide Haute Performance), montre qu'elle est constituée d'oligofructosanes de degré de polymérisation majoritairement compris entre 3 et 20.

Ce sont donc bien les oligofructosanes, en particulier les oligofructosanes de degré de polymérisation compris entre 3 et 20, qui confèrent au principe actif son efficacité.

### PROCEDE D'OBTENTION

Le principe actif selon l'invention tel que décrit précédemment est obtenu par un procédé comprenant une étape d'hydrolyse enzymatique.

Un procédé particulièrement adapté comprend au moins la succession des étapes suivantes :
- solubilisation de poudre de racine de *Cichorium intybus* dans l'eau,
- hydrolyse enzymatique,
- séparation des phases soluble et insoluble pour récupérer la phase soluble,
- inactivation enzymatique par traitement thermique,
- préférentiellement filtration et récupération du filtrat,
- purification et concentration de la fraction active,
- préférentiellement filtration et/ou filtration stérilisante et récupération du filtrat.

Les étapes de désodorisation et de décoloration peuvent être ajoutées.

Les paramètres des différentes étapes doivent être ajustés afin d'obtenir des principes actifs comprenant des oligofructosanes de degré de polymérisation compris entre 3 et 20, en particulier un principe actif dans lequel les oligofructosanes de degré de polymérisation compris entre 3 et 20 représentent au moins 69% en poids des sucres totaux du principe actif.

### COMPOSITIONS ET PROCEDE COSMETIQUE DE SOIN DE LA PEAU

La présente invention couvre aussi les compositions, en particulier les compositions cosmétiques, incluant un hydrolysat de racine de *Cichorium intybus* comprenant des oligofructosanes de degré de polymérisation compris entre 3 et 20 obtenu par un procédé comprenant une étape d'hydrolyse enzymatique, dans différentes formes galéniques, adaptées à l'administration par voie topique cutanée.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il peut s'agir de compositions comprenant entre 0,01 et 3% de principe(s) actif(s) issu(s) selon la présente invention.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur tels que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba ;
- les élastomères de silicone,
- les tensioactifs, de préférence émulsionnants, qu'ils soient non ioniques, anioniques, cationiques ou amphotères ;
- les co-tensioactifs, tels que les alcools gras linéaires ;
- les épaississants et/ou gélifiants,
- les humectants, tels que les polyols comme la glycérine ;
- les filtres organiques,
- les filtres inorganiques,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (International Cosmetic Ingrédient Dictionary and Handbook publié par le Personal Care Product Council).

Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à agir de façon similaire à la vitamine D sur les cellules de la peau, en particulier :
- à stimuler les voies de signalisation régulée par le récepteur à la vitamine D dans les cellules cutanées
- à stimuler la synthèse du récepteur à la vitamine D et/ou à augmenter la fonctionnalité du récepteur à la vitamine D dans les cellules cutanées
- à stimuler le réseau de gènes engagés dans la différenciation terminale des kératinocytes,
- à augmenter l'expression de KLF4, de la cytokératine 1, de l'involucrine, de la cystatine E/M et/ou de la KLK5 dans les cellules cutanées,
- à favoriser la formation de la barrière épidermique.

Ces compositions sont donc essentiellement destinées à préserver l'intégrité ou favoriser une récupération de la fonction barrière de la peau, en particulier sur des peaux susceptibles de présenter une déficience en vitamine D comme les peaux vieillies.

L'invention vise donc également à cet effet un procédé cosmétique de soin de la peau humaine, destiné à préserver l'intégrité ou favoriser une récupération de la fonction barrière de la peau, comprenant l'application topique d'une composition renfermant un hydrolysat de racine de *Cichorium intybus* comprenant des oligofructosanes de degré de polymérisation compris entre 3 et 20 obtenu par un procédé comprenant une étape d'hydrolyse enzymatique.

### EXEMPLES

Un exemple non limitatif de procédé d'obtention et de principe actif contenant des oligofructosanes de degré de polymérisation compris entre 3 et 20 obtenu par hydrolyse comprenant une étape d'hydrolyse enzymatique à partir de racine de *Cichorium intybus,* est présenté en suivant, ainsi que des exemples de composition incluant un tel principe actif.

### Exemple 1 : principe actif selon l'invention

Un exemple de procédé d'obtention d'un principe actif selon l'invention, comprend la mise en oeuvre des étapes suivantes :
- solubilisation de poudre de racine de *Cichorium intybus* dans l'eau à raison de 50g/l à 50°C pendant 3 heures,
- hydrolyse enzymatique à l'aide d'une carbohydrase,
- séparation des phases soluble et insoluble par décantation pour récupérer la phase soluble,
- inactivation enzymatique par traitement thermique,
- filtration et récupération du filtrat,
- purification et concentration de la fraction active,
- décoloration,
- filtration stérilisante.

Le principe actif obtenu présente les caractéristiques suivantes :
- aspect : liquide limpide
- couleur : jaune clair
- teneur en matières sèches : 60,8 g/l
- pH : 4,9
- teneur en sucres totaux : 46,3 g/l, soit 76,1% en poids par rapport à la matière sèche, dont 77% sous forme d'oligofructosanes
- teneur en protéines totales : 4,7% en poids par rapport à la matière sèche
- teneur en acides uroniques : 10,4% en poids par rapport à la matière sèche,
- teneur en cendres : 8,8% en poids par rapport à la matière sèche,
- teneur en polyphénols : 0,01% en poids par rapport à la matière sèche.

### Exemple 2 : utilisation d'un principe actif selon l'invention dans un gel matifiant

| Phase A . | Eau | QSP 100% |
|---|---|---|
| | Glycérol | 6% |
| | Carbopol Ultrez 20 | 0,5% |
| Phase B. | Lanol 1688 (Seppic) | 3% |
| | Montanov (Seppic) | 2% |
| | Sophiderm (Sophim) | 3% |
| | Patlac ISL (Rita) | 2% |
| | DUB 1632 (Stéarinerie Dubois) | 4% |
| | DUB MCT5545 (Stéarinerie Dubois) | 4% |
| Phase C. | Principe actif selon l'invention (exemple 1) | 3% |
| | Conservateur | 0,7% |
| Phase D. | NaOH | qsp pH6 |

Les quantités indiquées sont données en pourcentage de poids.

Ce gel émulsionné blanc compact présente un pH de 6. En application topique, il présente une application agréable et laisse un film doux.

Il peut être obtenu par la mise en oeuvre des étapes suivantes :
- mélanger A, et chauffer au bain marie à 80°C sous agitation magnétique en veillant à bien disperser le gel,
- mélanger B, et chauffer au bain-marie à 80°C sous agitation magnétique,
- émulsionner B dans A sous rotor sator à 2000 trs/min,
- à 60°*C*, ajouter *C* dans l'ordre, toujours sous rotor sator à 2000 trs/min,
- à 30°*C*, ajuster avec D, sous agitation mécanique à 1200 trs/min,
- laisser refroidir, sous agitation, pour contrôler l'homogénéisation de la crème.

### Exemple 3 : utilisation d'un principe actif selon l'invention dans une crème hydratante

| Phase A. | Eau | QSP 100 % |
|---|---|---|
| | Butylène glycol | 4,3% |
| Phase B. | Montanov 14 (Seppic) | 4% |
| | Alcool cétéarylique | 3% |
| | DUB SEG (Stéarinerie DUBOIS) | 3% |
| | DUB Lilirose (Stéarinerie DUBOIS) | 3% |
| | DUB Aprilose (Stéarinerie DUBOIS) | 3% |
| | Montanov 68 (Seppic) | 3% |
| Phase C. | Principe actif selon l'invention (exemple 1) | 3% |
| | Conservateur | 0,7 % |

Les quantités indiquées sont données en pourcentage de poids.

Cette émulsion blanche et compacte présente un pH de 6,5. En application topique, elle présente une application veloutée avec un fini doux et un effet homogène.

Elle peut être obtenue par la mise en oeuvre des étapes suivantes :
- mélanger A, chauffer au bain marie à 80°C sous agitation magnétique en veillant à bien disperser le gel,
- mélanger B, chauffer au bain-marie à 80°C sous agitation magnétique,
- émulsionner B dans A sous émulseur rotor stator à 3000 tours/minute,
- à 40°C, additionner C dans l'ordre sous rotor sator à 3000 tours/minute,
- laisser sous agitation jusqu'à complète homogénéisation.

### Exemple 4 : utilisation d'un principe actif selon l'invention dans une émulsion

| Phase A. | Eau | QSP 100% |
|---|---|---|
| | Glycérol | 6,7% |
| | Satialgine US 551 (Degussa) | 2% |
| Phase B. | Montanov 68 (Seppic) | 2% |
| | Montanov S (Seppic) | 1% |
| | Pelemol BB (Phoenix Chemical) | 2% |
| | Pelemol 2014 (Phoenix Chemical) | 3% |
| | Sophim MC30 (Sophim) | 3,5% |
| | DUB MCT 5545 (Stéarinerie Dubois) | 3% |
| | DUB Aprilose (Stéarinerie Dubois) | 2,6% |
| | DUB OK18 (Stéarinerie Dubois) | 3,5% |
| | DUN IPP (Stéarinerie Dubois) | 4% |
| Phase C. | Conservateur | 0,7 % |
| | Principe actif selon l'invention (exemple 1) | 3% |

Les quantités indiquées sont données en pourcentage de poids.

Ce gel émulsionné écru onctueux présente un pH de 5. En application topique, il présente une pénétration rapide avec un fini doux et sec.

Il peut être obtenu par la mise en oeuvre des étapes suivantes :
- mélanger A, disperser le gel au bain-marie à 80°C sous agitation magnétique à 1000 tours/minute,
- mélanger B, chauffer au bain-marie à 80°C sous agitation magnétique,
- émulsionner B dans A sous rotor-stator à 1700 tours/minute,
- à 40°C, ajouter C dans l'ordre indiqué sous rotor-sator à 1800 trs/min,
- A 30°C, ajouter lentement D pour ajuster le pH sous agitation entre 1000 et 1500 tours/minute et laisser agiter jusqu'à complète homogénéisation.

### Exemple 5 : utilisation d'un principe actif selon l'invention dans un sérum

| Phase A . | Eau | QSP 100% |
|---|---|---|
| | Carbopol 940 (Lubrizol) | 0,3% |
| | Glycérol | 6% |
| Phase B. | Simulsol 220 TM (Seppic) | 6,7% |
| | Stérol CC9595 (Alpinia) | 6,7% |
| | Eucarol D (Alpinia) | 3% |
| Phase C. | Principe actif selon l'invention (exemple 1) | 3% |
| | Conservateur | 0,7% |
| Phase C. | NaOH | qsp pH 7 |

Les quantités indiquées sont données en pourcentage de poids.

Ce gel émulsionné liquide transparent présente un pH de 7. En application topique, il présente une application confortable avec un fini sec et un effet mat.

Il peut être obtenu par la mise en oeuvre des étapes suivantes :
- mélanger A, chauffer au bain-marie à 80°C sous agitation magnétique en veillant à bien disperser le gel,
- mélanger B, chauffer au bain-marie à 80°C sous agitation magnétique,
- émulsionner B dans A sous rotor sator à 1000 tours/minute,
- à 30°C, ajouter C, dans l'ordre indiqué et ajuster le pH, avec D, lentement sous rotor sator à 1000 tours/minute et continuer l'agitation pour une complète homogénéisation.

### Exemple 6 : utilisation d'un principe actif selon l'invention dans une crème de jour

| Phase A. | Eau | QSP 100 % |
|---|---|---|
| | Butylène glycol | 3,5% |

| Phase B. | DC 73 100 (Dow Corning) | 4% |
|---|---|---|
| | Diamant N (Aiglon) | 4% |
| | Simulsol M45 (Seppic) | 4% |
| | DUB RG AE (Stéarinerie Dubois) | 3,5% |
| | Stéarate PEG 4000 (Stéarinerie Dubois) | 2% |
| | Lanol 14 M (Seppic) | 2% |
| | DUB MM (Stéarinerie DUBOIS) | 2% |
| | Pelemol 2014 (Phoenix Chemical) | 2% |
| | Montanov 14 (Seppic) | 3% |
| Phase C. | Principe actif selon l'invention (exemple 1) | 3% |
| | Conservateur | 0,7% |

Les quantités indiquées sont données en pourcentage de poids.

Cette émulsion blanche et fluide présente un pH de 4,7. En application topique, elle présente une pénétration aisée et filmogène.

Elle peut être obtenue par la mise en oeuvre des étapes suivantes :
- mélanger A, chauffer au bain marie à 80°C sous agitation magnétique,
- mélanger B, chauffer au bain-marie à 80°C sous agitation magnétique,
- émulsionner B dans A sous émulseur rotor stator à 1500 tours/minute,
- à 30°C, ajouter C dans l'ordre sous rotor sator à 1500 tours/minute, et poursuivre l'agitation jusqu'à complet refroidissement.

### Exemple 7 : utilisation d'un principe actif selon l'invention dans un gel émulsionné

| Phase A. | DUB GC7 (Stéarinerie Dubois) | 1,2% |
|---|---|---|
| | DUB MCT 5545 (Stéarinerie Dubois) | 0,8% |
| | CARBOPOL ETD2020 (Novéon) | 0,2% |
| | Conservateur | 0,7% |
| Phase B. | Principe actif selon l'invention (exemple 1) | 3% |
| Phase C. | NaOH | QS pH 6,3 |
| Phase D. | Eau | QSP 100% |

Les quantités indiquées sont données en pourcentage de poids.

Cette gel émulsionné présente un pH de 6,3. Il peut être obtenu par la mise en oeuvre des étapes suivantes :
- mélanger A et chauffer au bain marie à 45°C,
- homogénéiser A sous agitation mécanique jusqu'à complète dispersion du gel,
- ajouter B avec une pipette graduée,
- ajuster le pH lentement avec C avec une pastette en adaptant l'agitation à la viscosité du produit.

### EVALUATION DE L'EFFICACITE COSMETIQUE D'UN PRINCIPE ACTIF SELON L'INVENTION

### A. Tests in vitro

### I. Etude de l'effet sur la fonctionnalité du récepteur à la vitamine D

La vitamine D sous sa forme active (le calcitriol) étant centrale dans la régulation d'un réseau de gènes dédiés au contrôle de la différenciation épidermique terminale, il apparaît intéressant d'identifier la fonctionnalité du récepteur à la vitamine D (VDR) des peaux âgées par comparaison aux peaux jeunes et d'évaluer l'effet du principe actif selon l'invention sur les VDR.

Le premier objectif de cette étude est de comparer la synthèse de VDR entre des kératinocytes humains issus de donneurs jeunes(âge inférieur à 30 ans) et des kératinocytes issus de donneurs âgés (âge supérieur à 60 ans).

Le second objectif est d'évaluer l'effet d'un hydrolysat de racine de *Cichorium intybus* comprenant des oligofructosanes (exemple 1) sur sa capacité à augmenter la synthèse de VDR de kératinocytes humains issus de donneurs âgés (âge supérieur à 60 ans).

L'étude a été réalisée par Western Blot et le protocole opératoire est décrit en suivant.

A JO les kératinocytes humains de donneurs jeunes et de donneurs âgés sont ensemencés et incubés à 37°C.

A J2, les cellules sont traitées avec le principe actif de l'exemple 1 à 0,5%, 1% et 2% (V/V) ou avec le calcitriol à 10⁻⁷M (témoin positif). Les cellules sont ensuite incubées pendant 24 heures à 37°C.

A J3, les extraits cellulaires sont récupérés puis dosés par Western blot.

Les résultats obtenus sont présentés en suivant :

| | **Taux de VDR (UA)** | | **Taux de VDR / témoin jeunes (%)** | **Taux de VDR / témoin âgés (%)** |
|---|---|---|---|---|
| **Kératinocytes jeunes** | | | | |
| Témoin | 0,229 | | - | |
| Principe actif exemple 1 1% | 0,276 | | + 20 | |
| Principe actif exemple 1 2% | 0,293 | | +28 | |
| **Kératinocytes âgés** | | | | |
| Témoin | | 0,162 | - 29 | - |
| Calcitriol 10⁻⁷M | | 0,214 | | + 31 |
| Principe actif exemple 1 0,5% | | 0,196 | | + 21 |
| Principe actif exemple 1 1% | | 0,215 | | + 32 |
| Principe actif exemple 1 2% | | 0,221 | | + 36 |

Ces résultats montrent tout d'abord que la synthèse du récepteur à la vitamine D est significativement réduite (29% dans les conditions de cette étude) sur les kératinocytes humains âgés comparée à celle des kératinocytes humains jeunes.

En outre, on constate qu'un hydrolysat enzymatique de racine de *Cichorium intybus* comprenant des oligofructosanes de degré de polymérisation compris entre 3 et 20 permet d'augmenter la synthèse du récepteur à vitamine D sur kératinocytes humains. En particulier, sur kératinocytes humains âgés le principe actif de l'exemple 1 selon l'invention testé à 1% permet d'augmenter la synthèse du récepteur à la vitamine D de 32%.

Enfin, on constate également que l'hydrolysat enzymatique de racine de *Cichorium intybus* comprenant des oligofructosanes de degré de polymérisation compris entre 3 et 20 présente un effet similaire à celui obtenu avec le calcitriol.

### II. Etude de l'effet sur le réseau moléculaire impliqué dans la différenciation terminale des kératinocytes

Le but de cette étude est d'évaluer l'effet d'un hydrolysat enzymatique de racine de *Cichorium intybus* comprenant des oligofructosanes de degré de polymérisation compris entre 3 et 20 (exemple 1) sur sa capacité à moduler l'expression des ARNm codant pour des protéines de différenciation épidermique :
- Kruppel-Like Factor 4 (KLF4) : facteur de transcription impliqué dans la différenciation terminale des kératinocytes,
- Cytokératine 1 (CK1) : constituants des filaments intermédiaires de kératine,
- Involucrine : protéine de l'enveloppe cornée,
- Cystatine E/M : inhibiteur des cystéines protéases lysosomales intervenant dans la différenciation des kératinocytes et la desquamation,
- Kallikréine 5 (KLK5 ou SCTE) : *stratum corneum* tryptic enzyme impliquée dans la desquamation.

L'étude a été réalisée par PCR quantitative sur des kératinocytes humains normaux issus de donneurs âgés (âge supérieur à 60ans), selon le protocole opératoire décrit en suivant.

A J0 les kératinocytes humains de donneurs âgés sont ensemencés et incubés à 37°C.

A J2, les cellules sont traitées avec le principe actif de l'exemple 1 à 0,5%, 1% et 2% (V/V) ou avec le calcitriol à 10⁻⁷ M (témoin positif). Les cellules sont ensuite incubées pendant 48 heures à 37°C.

A J4, les cellules sont récupérées et les ARN totaux extraits. Les ARN sont reverse-transcripts et les ADN complémentaires obtenus analysés par PCR quantitative (Réaction en Chaîne par Polymérase quantitative).

La quantification de l'incorporation de fluorescence est mesurée en continu à l'aide d'un thermocycleur et la quantification relative est réalisée à l'aide d'un logiciel.

Les résultats obtenus sont présentés dans le tableau ci-après :

| | **Taux ARNm / Témoin (%)** | | | |
|---|---|---|---|---|
| | **Principe actif (exemple 1)** | | | **Calcitriol 10⁻⁷M** |
| | **0,5%** | **1%** | **2%** | |
| Cytokératine 1 | + 41 | + 72 | + 165 | 0 |
| Involucrine | + 40 | + 51 | + 81 | + 42 |
| KLF4 | + 8 | + 22 | + 8 | + 25 |
| Cystatine E/M | + 29 | + 41 | + 57 | + 65 |
| KLK5 | + 19 | + 22 | + 23 | + 26 |

Ces résultats montrent que sur kératinocytes humains âgés, un hydrolysat de racine de *Cichorium intybus* comprenant des oligofructosanes obtenu par un procédé comprenant une étape d'hydrolyse enzymatique augmente significativement l'expression des gènes impliqués dans la différenciation terminale épidermique.

On constate également que l'effet de l'hydrolysat de racine de *Cichorium intybus* comprenant des oligofructosanes obtenu par un procédé comprenant une étape d'hydrolyse enzymatique est comparable à celui obtenu avec le calcitriol.

### III. Etude de l'effet sur la formation de la barrière cutanée

Le but de cette étude est d'évaluer la capacité d'un hydrolysat de racine de *Cichorium intybus* comprenant des oligofructosanes obtenu par un procédé comprenant une étape d'hydrolyse enzymatique à favoriser la construction d'un épiderme stratifié et fonctionnel à partir de kératinocytes humains vieillis obtenus par réplications successives.

Le protocole opératoire est décrit en suivant.

Au préalable, les kératinocytes humains jeunes (Passage 1 : P1) sont cultivés et répliqués jusqu'au passage 5 (P5) dans un milieu de culture spécifique (CnT-57) contenant ou pas, le principe actif de l'exemple 1 à 0,5% ou le calcitriol à 10⁻⁸M (contrôle positif).

Ensuite, les Epidermes Reconstruits (ER) sont obtenus après culture des kératinocytes humains jeunes ou vieillis.

A J12 et J19, les ER sont récupérés, fixés, déshydratés et inclus en paraffine. Des coupes (4µm) sont ensuite réalisées à l'aide d'un microtome.

On réalise ensuite une coloration HE, puis un marquage immunohistologique de la filaggrine, marqueur de différenciation terminale.

La visualisation est réalisée sur microscope couplé à un système d'analyse d'images :
- l'épaisseur des épidermes (couches cellulaires vivantes) est mesurée sur les coupes histologiques après la coloration HE,
- la synthèse de la filaggrine est proportionnelle à l'intensité de la fluorescence (couleur verte) présente sur les épidermes reconstruits.

Les résultats obtenus sont présentés dans les deux tableaux ci-après ainsi sur les figures la à 1d et 2a à 2d pour les résultats à J19:

| | **Epaisseur des épidermes (µm)** | |
|---|---|---|
| | **J12** | **J19** |
| **Epidermes reconstruits jeunes** | | |
| Témoin | 46,4 | 72,6 (figure 1a) |

| **Epidermes reconstruits vieillis** | | |
|---|---|---|
| Témoin | 29,6 | 43,2 (figure 1b) |
| Calcitriol 10⁻⁸M | 37,8 | 58,6 (figure 1c) |
| Principe actif exemple 1 0,50% | 40,2 | 54,2 (figure 1d) |

| | **Synthèse de filaggrine (× 10⁴ UA)** | |
|---|---|---|
| | **J12** | **J19** |
| **Epidermes reconstruits jeunes** | | |
| Témoin | 137 | 1472 (figure 2a) |

| **Epidermes reconstruits vieillis** | | |
|---|---|---|
| Témoin | 41 | 354 (figure 2b) |
| Calcitriol 10⁻⁸M | 116 | 795 (figure 2c) |
| Principe actif exemple 1 0,50% | 196 | 897 (figure 2d) |

On constate tout d'abord que la qualité et la fonctionnalité des épidermes construits à partir de kératinocytes humains vieillis sont altérées par rapport à celles des épidermes construits à partir de kératinocytes humains jeunes.

On constate également que le prétraitement des kératinocytes vieillis avec un hydrolysat de racine de *Cichorium intybus* comprenant des oligofructosanes de degré de polymérisation compris entre 3 et 20 obtenu par un procédé comprenant une étape d'hydrolyse enzymatique à 0,50% permet d'augmenter l'épaisseur des épidermes et la synthèse de filaggrine.

Ces résultats montrent donc bien qu'un hydrolysat de racine de *Cichorium intybus* comprenant des oligofructosanes de degré de polymérisation compris entre 3 et 20 obtenu par un procédé comprenant une étape d'hydrolyse enzymatique améliore la capacité des kératinocytes humains vieillis à construire un épiderme stratifié et fonctionnel.

### IV. Etude de l'activité antiradicalaire

Cette étude a pour objectif d'évaluer l'activité anti-radicalaire d'un hydrolysat enzymatique de racine de *Cichorium intybus* de degré de polymérisation compris entre 3 et 20 obtenu par un procédé comprenant une étape d'hydrolyse enzymatique comprenant des oligofructosanes de degré de polymérisation compris entre 3 et 20 obtenu par un procédé comprenant une étape d'hydrolyse enzymatique, selon la méthode au DPPH (Di Phényl Picrylhydrazyl Hydrate).

Le DPPH est un radical libre, absorbant dans le violet à 517nm. Un produit anti-radicalaire entraîne une disparition de la coloration violette.

La solution DPPH utilisée pour cette étude est une solution préparée à partir de 4,8mg de DPPH dissout dans du méthanol.

Le protocole de l'étude est décrit en suivant.

On ajoute dans des tubes à hémolyse de la solution DPPH et le principe actif de l'exemple 1 à 10% et 20% ou de l'eau distillée (pour le témoin).

On agite et on attend 20 minutes avant de lire les densités optiques à 517nm contre l'air.

Les résultats obtenus sont présentés en pourcentage d'activité anti-radicalaire, dans le tableau ci-dessous :

| Dose de principe actif exemple 1 | Activité anti-radicalaire |
|---|---|
| 10% | 0% |
| 20% | 1,6% |

Ces résultats montre qu'un principe actif obtenu par hydrolyse de racine de *Cichorium intybus* comprenant des oligofructosanes de degré de polymérisation compris entre 3 et 20 ne présente pas d'activité anti-radicalaire selon la méthode au DPPH.

### B. Tests in vivo

### I. Etude de la capacité à renforcer la barrière cutanée

L'objectif de cette étude est de quantifier *in vivo,* sur volontaires, l'effet d'un hydrolysat de racine de *Cichorium intybus* comprenant des oligofructosanes de degré de polymérisation compris entre 3 et 20 obtenu par un procédé comprenant une étape d'hydrolyse enzymatique (exemple 1) formulé à 3% en gel-émulsionné (composition de l'exemple 8) sur la Perte Insensible en Eau (PIE) de la peau.

Cet effet a été observé après 7 et 14 jours d'applications biquotidiennes et comparé au placebo après une perturbation artificielle chronique de la fonction barrière à l'aide d'un détergent le Lauryl Sulfate de Sodium (SLS) utilisé à 10%.

En effet, la barrière cutanée joue un rôle régulateur dans l'équilibre en eau de la peau. Lorsque celle-ci est lésée, il apparaît des dérèglements dans la régulation des échanges d'eau. L'eau migre alors plus facilement vers le milieu extérieur ce qui augmente la perte insensible en eau. En revanche, si l'état de la barrière cutanée s'améliore, les valeurs de pertes en eau vont diminuer car la régulation des échanges en eau sera assurée de façon correcte.

L'étude a été réalisée en période hivernale sur 20 volontaires sélectionnés selon les critères suivants :
- âge moyen supérieur à 65 ans (les adultes âgés étant plus disposés à présenter une déficience en vitamine D)
- sexe féminin (les femmes sont une population plus à risque de déficience en vitamine D)
- ne prenant pas de complément alimentaire contenant de la vitamine D ou de supplémentation en vitamine D, et
- ayant une peau normale au niveau des bras.

Les mesures de la perte insensible en eau ont été réalisées à l'aide d'un Tewamètre®, dispositif muni d'une sonde qui mesure le gradient de vapeur d'eau se mettant en place entre la surface cutanée et l'air ambiant.

Le protocole opératoire de l'étude est le suivant.

Entre J-14 et J0, les volontaires n'appliquent aucune crème au niveau du haut des bras.

A J0, on détermine trois zones de mesure au niveau du haut des bras :
- Zone non traitée
- Zone placebo
- Zone traitée avec le principe actif de l'exemple 1 formulé selon l'exemple 8.

Des mesures de la PIE sont réalisées sur chaque zone à l'aide d'un Tewamètre®. Entre J0 et J6 :
- les zones étudiées sont lavées avec un savon irritant (SLS) qui permet d'augmenter les pertes en eau,
- les produits sont appliqués biquotidiennement sur les zones dédiées.

A J7, des mesures de la PIE sont réalisées sur chaque zone au Tewamètre®.

Entre J7 et J13 :
- les zones étudiées sont lavées avec un savon irritant (SLS) qui permet d'augmenter les pertes en eau,
- les produits sont appliqués biquotidiennement sur les zones dédiées.

A J14, des mesures de la PIE sont réalisées sur chaque zone au Tewamètre®. Les résultats obtenus pour le principe actif selon l'invention en pourcentage par rapport aux résultats obtenus avec le placebo sont présentés dans le tableau suivant :

| | Variation / Placebo (%) |
|---|---|
| J7 | -14,3% |
| J14 | -16,9% |

Ces résultats montrent que dans les conditions de cette étude, après 7 jours d'applications biquotidiennes et en comparaison au placebo, un hydrolysat de racine de *Cichorium intybus* comprenant des oligofructosanes de degré de polymérisation compris entre 3 et 20 obtenu par un procédé comprenant une étape d'hydrolyse enzymatique selon l'invention formulé à 3% en gel-émulsionné diminue significativement de 14,3% la perte insensible en eau après agression répétée au SLS. Cet effet est également mesuré après 14 jours d'étude avec une diminution significative de la PIE de 16,9%.

L'utilisation d'un hydrolysat de racine de *Cichorium intybus* comprenant des oligofructosanes de degré de polymérisation compris entre 3 et 20 obtenu par un procédé comprenant une étape d'hydrolyse enzymatique, permet donc de limiter l'altération de la fonction barrière et renforcer ainsi la barrière cutanée chez des personnes matures de plus de 65 ans susceptibles de présenter une déficience en vitamine D.

### II. Etude de la capacité à accélérer la récupération de la fonction barrière

L'objectif de cette étude est d'évaluer *in vivo* sur volontaires, l'efficacité d'un hydrolysat de racine de *Cichorium intybus* comprenant des oligofructosanes de degré de polymérisation compris entre 3 et 20 obtenu par un procédé comprenant une étape d'hydrolyse enzymatique (exemple 1) formulé à 3% en gel-émulsionné (composition de l'exemple 8), sur la capacité de récupération de la fonction barrière après une agression unique à l'aide d'un patch contenant du Lauryl Sulfate de Sodium (SLS). Cet effet a été évalué après 7 et 14 jours d'applications biquotidiennes en mesurant la Perte Insensible en Eau (PIE) de la peau.

L'étude a été réalisée en période hivernale sur 19 volontaires sélectionnés selon les critères suivants :
- âge moyen supérieur à 65 ans (les adultes âgés étant plus disposés à présenter une déficience en vitamine D)
- sexe féminin (les femmes sont une population plus à risque de déficience en vitamine D)
- ne prenant pas de complément alimentaire contenant de la vitamine D ou de supplémentation en vitamine D, et
- ayant une peau normale au niveau des bras.

Les mesures de la perte insensible en eau ont été réalisées à l'aide d'un Tewamètre®, dispositif muni d'une sonde qui mesure le gradient de vapeur d'eau se mettant en place entre la surface cutanée et l'air ambiant.

Le protocole opératoire de l'étude est le suivant.

Entre J-14 et J0, les volontaires n'appliquent aucune crème au niveau du haut des bras.

A J_{avant agression}, on détermine trois zones de mesure au niveau du haut des bras :
- Zone non traitée
- Zone placebo
- Zone traitée avec le principe actif de l'exemple 1 formulé selon l'exemple 8.

Des mesures de la PIE sont réalisées sur chaque zone à l'aide d'un Tewamètre®. Un Patch occlusif de SLS est appliqué à 0,8% sur chacune des trois zones. 24 heures après la pose, les patchs sont retirés.

A J0, 72 heures après le retrait des patchs, on repère les zones de mesure au niveau des avant-bras, et des mesures de la PIE sont réalisées sur chaque zone à l'aide d'un Tewamètre®.

Entre J0 et J14, les produits (principe actif et placebo) sont appliqués biquotidiennement sur les zones dédiées.

A J2,J3,J4,J7,J9,J11 et J14, des mesures de la PIE sont réalisées sur chaque zone au Tewamètre®.

Les résultats obtenus sont présentés dans le tableau suivant :

| | **Récupération de la fonction barrière (%)** | | | | | | |
|---|---|---|---|---|---|---|---|
| | J2 | J3 | J4 | J7 | J9 | J11 | J14 |
| Zone non traitée | 30% | 45% | 49% | 67% | 72% | 83% | 92% |
| Placebo | 30% | 44% | 50% | 68% | 75% | 82% | 91% |
| Gel émulsionné exemple 8 | 36% | 51% | 58% | 72% | 80% | 88% | 94% |
| | | | | | | | |
| gel émulsionné exemple 8 / placebo | *0,1809* | *0,0768* | *0,0072* | *0,0461* | *0,0324* | *0,0090* | *0,4124* |

On constate que dans les conditions de cette étude, après 14 jours d'applications biquotidiennes et en comparaison au placebo, un hydrolysat de racine de *Cichorium intybus* comprenant des oligofructosanes de degré de polymérisation compris entre 3 et 20 obtenu par un procédé comprenant une étape d'hydrolyse enzymatique formulé à 3% en gel-émulsionné, favorise une récupération significativement plus rapide de la fonction barrière après une agression unique et préalable au SLS. Dès quatre jours d'application, les pertes en eau sont diminuées de 58%. Ces résultats montrent donc bien qu'un hydrolysat de racine de *Cichorium intybus* comprenant des oligofructosanes de degré de polymérisation compris entre 3 et 20 obtenu par un procédé comprenant une étape d'hydrolyse enzymatique formulé à 3% accélère, après une agression, le retour à une fonction barrière normale et améliore ainsi la capacité de récupération de la peau, chez des personnes matures susceptibles de présenter une déficience en vitamine D.

## Revendications

1. Principe actif destiné à une utilisation dans une composition à application cutanée **caractérisé en ce qu'**il s'agit d'un hydrolysat de racine de *Cichorium intybus* comprenant des oligofructosanes de degré de polymérisation compris entre 3 et 20 obtenu par un procédé comprenant une étape d'hydrolyse enzymatique

2. Principe actif selon l'une des précédentes revendications, **caractérisé en ce qu'**il comprend au moins 46% de sucres totaux par rapport au poids total de matière sèche.

3. Principe actif selon l'une des précédentes revendications, caractérisé en que les oligofructosanes représentent au moins 69% des sucres totaux présents dans le principe actif.

4. Principe actif selon l'une des précédentes revendications, **caractérisé par** au moins l'une des caractéristiques suivantes :
- un taux de matières sèches compris entre 30 et 90 g/l,
- une teneur en sucres totaux comprise entre 20 et 62 g/l.

5. Principe actif selon l'une des précédentes revendications, **caractérisé par** au moins l'une des caractéristiques suivantes :
- un taux de matières sèches compris entre 45 et 65 g/l,
- une teneur en sucres totaux comprise entre 30 et 45 g/l.

6. Principe actif selon l'une des précédentes revendications, pour son application comme principe actif dans une composition à application cutanée, ledit principe actif et/ou ladite composition étant destinés à agir de façon similaire à la vitamine D sur les cellules de la peau.

7. Principe actif selon l'une des précédentes revendications, pour son application comme principe actif dans une composition à application cutanée, ledit principe actif et/ou ladite composition étant destinés à stimuler les voies de signalisation régulée par le récepteur à la vitamine D dans les cellules cutanées.

8. Principe actif selon l'une des précédentes revendications, pour son application comme principe actif dans une composition à application cutanée, ledit principe actif et/ou ladite composition étant destinés à stimuler la synthèse du récepteur à la vitamine D et/ou à augmenter la fonctionnalité du récepteur à la vitamine D dans les cellules cutanées.

9. Principe actif selon l'une des précédentes revendications, pour son application comme principe actif dans une composition à application cutanée, ledit principe actif et/ou ladite composition étant destinés à stimuler le réseau de gènes engagés dans la différenciation terminale des kératinocytes.

10. Principe actif selon l'une des précédentes revendications, pour son application comme principe actif dans une composition à application cutanée, ledit principe actif et/ou ladite composition étant destinés à augmenter l'expression de KLF4, de la cytokératine 1, de l'involucrine, de la cystatine E/M et/ou de la KLK5 dans les cellules cutanées.

11. Principe actif selon l'une des précédentes revendications, pour son application comme principe actif dans une composition à application cutanée, ledit principe actif et/ou ladite composition étant destinés à favoriser la formation de la barrière épidermique.

12. Principe actif selon l'une des revendications 6 à 11, pour son application comme principe actif dans une composition à application cutanée, ledit principe actif et/ou ladite composition étant destinés à être appliqués sur des peaux susceptibles de présenter une déficience en vitamine D.

13. Principe actif selon la revendication 12, pour son application comme principe actif dans une composition à application cutanée, ledit principe actif et/ou ladite composition étant destinés à être appliqués sur des peaux âgées, vieillies.

14. Composition cosmétique pour application topique, **caractérisée en ce qu'**elle comprend un principe actif selon l'une des revendications 1 à 13 présent entre 0,01 et 3% en poids total de la composition.

15. Procédé cosmétique non-thérapeutique de soin de la peau humaine, destiné à préserver l'intégrité ou favoriser une récupération de la fonction barrière de la peau, comprenant l'application sur la peau d'une composition selon la revendication 14.

## Patentansprüche

1. Wirkstoff, der zu einer Verwendung in einer Zusammensetzung zur kutanen Anwendung bestimmt ist, **dadurch gekennzeichnet, dass** es sich um ein Hydrolysat der Wurzel von *Cichorium intybus* handelt, das Oligofruktane mit einem Polymerisationsgrad zwischen 3 und 20 aufweist und das durch ein Verfahren hergestellt ist, das einen Verfahrensschritt mit einer enzymatischen Hydrolyse aufweist.

2. Wirkstoff nach einem der vorhergehenden Ansprüche, **dadurch gekennzeichnet, dass** dieser wenigstens 46% Gesamtzucker bezüglich der gesamten Trockenmasse aufweist.

3. Wirkstoff nach einem der vorhergehenden Ansprüche, **dadurch gekennzeichnet, dass** die Oligofruktane wenigstens 69% des im Wirkstoff vorliegenden Gesamtzuckers ausmachen.

4. Wirkstoff nach einem der vorhergehenden Ansprüche, **gekennzeichnet durch** wenigstens eine der folgenden Eigenschaften:
- eine Trockenmasse zwischen 30 und 90 g/l,
- einen Gesamtzuckergehalt zwischen 20 und 62 g/l.

5. Wirkstoff nach einem der vorhergehenden Ansprüche, **gekennzeichnet durch** wenigstens eine der folgenden Eigenschaften:
- eine Trockenmasse zwischen 45 und 65 g/l,
- einen Gesamtzuckergehalt zwischen 30 und 45 g/l.

6. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, auf die Hautzellen auf ähnliche Weise wie Vitamin D einzuwirken.

7. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, die Signalisierungspfade anzuregen, die durch den Rezeptor von Vitamin D in den Hautzellen gesteuert sind.

8. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, die Synthese des Rezeptors für Vitamin D anzuregen und/oder die Funktionalität des Rezeptors für Vitamin D in den Hautzellen zu verbessern.

9. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, das Netz von Genen anzuregen, die an der abschließenden Differenzierung von Keratinozyten beteiligt sind.

10. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, die Expression von KLF4, von Cytokeratin 1, von Involucrin, von Cystatin E/M und/oder KLK5 in Hautzellen zu verbessern.

11. Wirkstoff nach einem der vorhergehenden Ansprüche zur Anwendung als Wirkstoff in einer Zusammensetzung zur kutanen Anwendung, wobei der Wirkstoff und/oder die Zusammensetzung dazu bestimmt sind, die Bildung von Hautbarrieren zu fördern.

12. Wirkstoff nach einem der Ansprüche 6 bis 11 zur Anwendung als Wirkstoff in einer Zusammensetzung zur kutanen Anwendung, wobei der wirkstoff und/oder die Zusammensetzung dazu bestimmt sind, auf einer Haut angewendet zu werden, die dazu neigt, einen Mangel an Vitamin D zu zeigen.

13. Wirkstoff nach Anspruch 12 zur Anwendung als Wirkstoff in einer Zusammensetzung zur kutanen Anwendung, wobei der Wirkstoff und/oder die Zusammensetzung dazu bestimmt ist, auf eine betagte oder gealterte Haut angewendet zu werden.

14. Kosmetische Zusammensetzung zur topischen Anwendung, **dadurch gekennzeichnet, dass** diese einen Wirkstoff nach einem der Ansprüche 1 bis 13 aufweist, der in 0,01 - 3% des Gesamtgewichts der Zusammensetzung vorliegt.

15. Kosmetisches nichttherapeutisches Verfahren zur Pflege der menschlichen Haut, das dazu bestimmt ist, die Unversehrtheit der Haut zu bewahren oder die Funktion der Hautbarriere wiederherzustellen und das die Anwendung einer Zusammensetzung nach Anspruch 14 auf der Haut umfasst.

## Claims

1. An active principle intended for use in a composition for skin application, **characterised in that** it is a hydrolysate of *Cichorium intybus* root comprising oligofructosans with a degree of polymerisation between 3 and 20, obtained by a method comprising an enzymatic hydrolysis step.

2. An active principle according to any of the preceding claims, **characterised in that** it comprises at least 46% total sugars with respect to the total weight of dry matter.

3. An active principle according to any of the preceding claims, **characterised in that** the oligofructosans represent at least 69% of the total sugars present in the active principle.

4. An active principle according to any of the preceding claims, **characterised by** at least one of the following features:
- a proportion of dry matter of between 30 and 90 g/l,
- a total sugar content of between 20 and 62 g/l.

5. An active principle according to any of the preceding claims, **characterised by** at least one of the following features:
- a proportion of dry matter of between 45 and 65 g/l,
- a total sugar content of between 30 and 45 g/l.

6. An active principle according to any of the preceding claims, for application thereof as an active principle in a composition for skin application, said active principle and/or said composition being intended to act on the skin cells in a similar fashion to vitamin D.

7. An active principle according to any of the preceding claims, for application thereof as an active principle in a composition for skin application, said active principle and/or said composition being intended to stimulate the signalling channels regulated by the vitamin D receptor in the skin cells.

8. An active principle according to any of the preceding claims, for application thereof as an active principle in a composition for skin application, said active principle and/or said composition being intended to stimulate the synthesis of the vitamin D receptor and/or to increase the functionality of the vitamin D receptor in the skin cells.

9. An active principle according to any of the preceding claims, for application thereof as an active principle in a composition for skin application, said active principle and/or said composition being intended to stimulate the gene network engaged in the terminal differentiation of keratinocytes.

10. An active principle according to any of the preceding claims, for application thereof as an active principle in a composition for skin application, said active principle and/or said composition being intended to increase the expression of KLF4, cytokeratin 1, involucrin, cystatin E/M and/or KLK5 in the skin cells.

11. An active principle according to any of the preceding claims, for application thereof as an active principle in a composition for skin application, said active principle and/or said composition being intended to promote the formation of the epidermal barrier.

12. An active principle according to any of claims 6 to 11, for application thereof as an active principle in a composition for skin application, said active principle and/or said composition being intended to be applied to skins liable to have a vitamin D deficiency.

13. An active principle according to claim 12, for application thereof as an active principle in a composition for skin application, said active principle and/or said composition being intended to be applied to old or aged skins.

14. A cosmetic composition for topical application, **characterised in that** it comprises an active principle according to any of claims 1 to 13 present at between 0.01% and 3% by total weight of the composition.

15. A non-therapeutic cosmetic method for human skin care, intended to preserve the integrity or promote the recovery of the skin barrier function, comprising the application to the skin of a composition according to claim 14.
